(19) Europäisches Patentamt
European Patent Office
Office européen des brevets

(11) Numéro de publication: **0 305 264 B1**

# (12) FASCICULE DE BREVET EUROPEEN

(45) Date de publication de fascicule du brevet: **03.03.93**

(51) Int. Cl.[5]: **C07F 9/00**, C07C 323/27, A61K 31/28

(21) Numéro de dépôt: **88402084.3**

(22) Date de dépôt: **10.08.88**

## (54) Composé organo-minéral de vanadyle, procédé d'obtention d'un tel composé, composition pharmaceutique contenant ce composé organo-minéral.

(30) Priorité: **11.08.87 FR 8711425**

(43) Date de publication de la demande:
**01.03.89 Bulletin 89/09**

(45) Mention de la délivrance du brevet:
**03.03.93 Bulletin 93/09**

(84) Etats contractants désignés:
**AT BE CH DE ES GB GR IT LI LU NL SE**

(56) Documents cités:
**US-A- 3 076 830**

**CHEMICAL ABSTRACTS, vol. 94, no. 16, 20 avril 1981, page 678, résumé no. 131408c, Columbus, Ohio, US; H. SAKURAI et al.: "Cysteine methyl ester-oxovanadium (IV) complex, preparation and characterization", & INORG. CHIM. ACTA 1980, 46(6), L119-L120**

(73) Titulaire: **PANMEDICA S.A.**
**1ère avenue-2065 M - L.I.D.**
**F-06516 Carros(FR)**

(72) Inventeur: **Lazaro, René**
**428 rue de Baillarguet**
**F-34170 Clapiers(FR)**
Inventeur: **Cros, Gérard**
**145 B, Impasse de la Voie Romaine**
**F-34090 Montpellier(FR)**
Inventeur: **McNeill, John H.**
**1238 Pacific Drive**
**Delta B.C. V4M2K6(CA)**
Inventeur: **Serrano, Jean-Jacques**
**999, Avenue d'Occitanie**
**F-34090 Montpellier(FR)**

(74) Mandataire: **Ores, Irène et al**
**CABINET ORES 6, Avenue de Messine**
**F-75008 Paris (FR)**

EP 0 305 264 B1

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition (art. 99(1) Convention sur le brevet européen).

## Description

La présente invention concerne généralement un composé organo-minéral de vanadyle. Elle a essentiellement pour objet un complexe comprenant du vanadyle, des procédés d'obtention d'un tel complexe et uner composition pharmaceutique contenant le complexe.

L'invention trouve notamment application dans l'industrie pharmaceutique, pour la fabrication de médicaments antidiabétiques, administrables par voie orale.

De nombreux composés sont connus pour leur activité antidiabétique chez des personnes souffrant de diabète insulino-dépendsnt. Mais, les compositions classiques à base de ces composés sont administrées par voie parentérale.

On a découvert quelques composés qui sont administrés par voie orale. On connait, par exemple, des composés à base de vanadate qui est la forme oxydée du vanadium, tel que le vanadate de sodium qui, administré oralement, a un effet antidiabétique. En effet, dans "Clayton, Arun, et Mc Neill, SCIENCE, 22 Mars 1985, Vol.227, pp 1474-1477", Mc Neill et autres ont démontré qu'en administrant du vanadate de sodium dans l'eau de boisson chez les rats diabétiques, on normalise la glycémie de ces derniers et on prévient l'altération de la fonction cardiaque associée au diabète.

Mais, le vanadate de sodium présente une toxicité digestive de par sa très faible absorption qui est de l'ordre de 1 à 3%. Le vanadate ($V^{5+}$) possède de nombreuses propriétés dont certaines entraînent cette toxicité non digestive, comme par exemple la propriété d'inhiber l'enzyme $Na^{+}/K^{+}$ ATPase dans les mécanismes d'absorption.

Par ailleurs, il est connu qu'au niveau intracellulaire, le vanadium manifeste son activité antidiabétique sous la forme vanadyle ($V^{4+}$) en favorisant ou en induisant la phosphorylation du récepteur à insuline.

Or, le sulfate de vanadyle $VOSO_4$ administré oralement à des rats diabétiques a bien un effet antidiabétique mais présente une toxicité aigüe.

D'autres composés de vanadium ont également été décrits : le Brevet américain n° 3 076 830 (J.B. CONN) décrit des complexes d'oxyde de vanadium ou vanadyle et d'un résidu acide d'un amino $\alpha$- ou $\beta$-hydroxyacide ou de thioacides, dans lesquels à la fois le groupement amine et le groupement carboxyle ne sont pas substitués, lesquels produits étant de plus utilisés en tant qu'inhibiteurs de la synthèse du cholestérol ; le résumé publié dans "Chemical Abstracts, 1981, vol. 94, n° 16, p. 678, n° 131408c, au nom de SAKURAI et al., décrit un complexe de vanadyle et d'ester méthylique de cystéine.

La présente invention a pour objet de fournir un nouveau composé organo-minéral de vanadyle ne présentant pas les inconvénients précités dans le cas d'utilisation du vanadate de sodium et du sulfate de vanadyle, et qui est à la fois non toxique et actif comme composé anti-diabétique. Un tel composé, demeurant sous la forme $V^{4+}$ au niveau sanguin, est mieux absorbé au niveau digestif et peut diffuser au niveau tissulaire sous uné forme active.

La solution conforme à la présente invention, pour résoudre ce problème, consiste en un composé organo-minéral de vanadyle se présentant sous la forme d'un complexe de vanadyle et de cystéïne. Ce composé a la formule générale suivante :

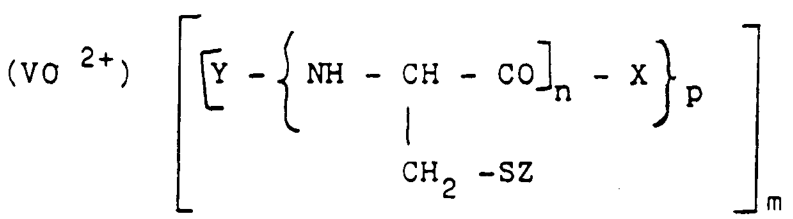

(I)

dans laquelle n, p et m sont respectivement des nombres entiers ayant pour valeur 1 ou 2 et

* dans le cas où p = 1 :

- Y représente un atome d'hydrogène, un groupe $-CH_2-$ ou un groupe R'-CO, dans lequel R' est un groupe alkyle, aryle ou aralkyle ;
- Z représente une charge - ou un atome d'hydrogène et
  (a) lorsque n = 1 et m = 2, X représente un groupe $O^-$, un groupe OR ou un groupe NHR, R étant un groupe aryle, aralkyle ou alkyle, à l'exclusion du groupe méthyle, à condition que :
  lorsque X représente un groupe $O^-$,
  Z représente un atome d'hydrogène et
  Y représente un groupe R'-CO et

lorsque X représente un groupe OR ou un groupe NHR,
Z représente une charge - et
Y représente un groupe $-CH_2-$ ou un atome d'hydrogène
et
(b) lorsque n = 2 et m = 1, X représente l'un des groupes suivants :

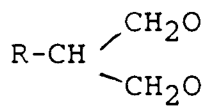
,
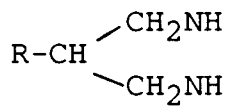

ou
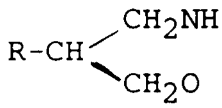

R étant un groupe aryle ou aralkyle ou un groupe alkyle, à l'exclusion du groupe méthyle et
Z représente une charge et
Y représente un atome d'hydrogène et
* dans le cas où p = 2
- n = 1
- m = 1
- X représente un groupe $O^-$,
- Z représente un atome d'hydrogène et
- Y représente
un groupe

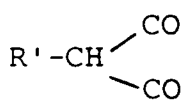
ou

un groupe

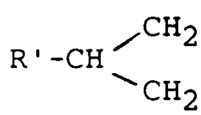
,

R' étant un groupe alkyle, aryle ou aralkyle.

Un tel composé, de par la complexation de l'ion vanadyle par un dérivé de la cystéine offre une activité anti-diabétique supérieure et une toxicité inférieure à celles des composés anti-diabétiques, administrés oralement, connus dans l'état de la technique.

Dans la formule précitée, lorsque p est égal à 1, Y correspond à un hydrogène et Z correspond à une charge -, un tel complexe présente un groupement amine libre.

Dans la formule précitée, lorsque n ou p est égal à 1 et m est égal à 2, le complexe se présente alors sous la forme d'un ligand bimoléculaire.

Dans la formule précitée, lorsque n ou p est égal à 2 et m est égal à 1, le complexe se présente alors sous la forme d'un ligand monomoléculaire.

Dans la formule précitée, lorsque n est égal à 1, X correspond à un groupement $O^-$ et Z correspond à un hydrogène ; ce second type de complexe présente un groupement carboxyle libre.

Selon une particularité de l'invention, dans la formule précitée, lorsque n est égal à 1 et p est égal à 1 et m est égal à 2, ce composé est un ligand monomoléculaire avec des groupements amine et carboxyle substitués.

Conformément à l'invention, la cystéine est sous sa forme L ou sous sa forme D.

Selon un mode de réalisation avantageux du composé conforme à l'invention, p = 1, n = 1, m = 2, X représente un groupe OR ou un groupe NHR dans lequel R représente un groupe aryle, aralkyle ou alkyle, a l'exclusion du groupe méthyle, lequel composé correspond à l'une quelconque des formules II ou VI :

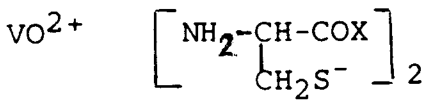

(II)

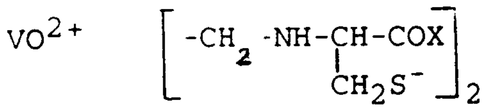

(VI)

Selon un autre mode de réalisation avantageux du composé conforme à l'invention, p = 1, n = 1, m = 2, Y représente un groupe R'-CO dans lequel R' est un groupe alkyle, aryle ou aralkyle, lequel composé correspond à la formule IV suivante :

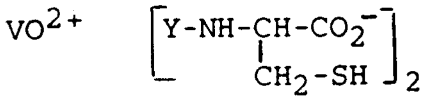

(IV)

Selon un autre mode de réalisation avantageux du composé conforme à l'invention, p = 1, n = 2, m = 1, et X représente l'un des groupes suivants :

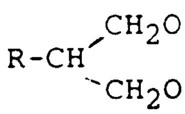 , 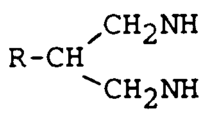 ou 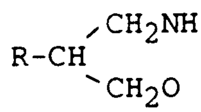

dans lesquels

R est un groupe aryle, aralkyle ou alkyle, à l'exclusion du groupe méthyle,

lequel composé correspond à la formule III suivante :

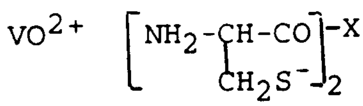

(III)

Selon un autre mode de réalisation avantageux du composé conforme à l'invention, p = 2, n = 1, m = 1, et Y représente

un groupe

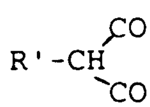

ou un groupe

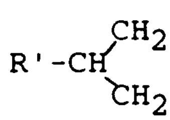 ,

dans lesquels

R' est un groupe alkyle, aryle ou aralkyle, lequel composé correspond à la formule V suivante :

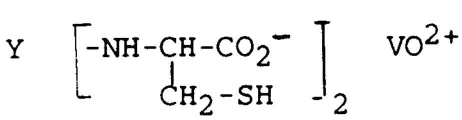

(V)

Un autre objet de l'invention consiste en un procédé de préparation du composé organo-minéral de vanadyle se présentant sous la forme d'un complexe de vanadyle et de cystéine de formule (I) et comprenant un groupement amine libre ou des groupements amine et carboxyle substitués.

Un tel procédé est caractérisé en ce qu'il consiste à :

(a) préparer, au cours d'une première étape, un dérivé de cystéine, en faisant réagir une amine monoou bifonctionnelle ou un alcool mono- ou bifonctionnel, ou une amine-alcool avec la cystéine ou un dérivé de celle-ci, protégé sur la fonction amine et la fonction thiol par un groupement tertiobutyloxycarbonyl en présence de dicyclohexyl carbodiimide/hydroxybenzotriazole, et en éliminant ledit groupement tertiobutyloxycarbonyl par acidolyse et dans une seconde étape

(b) ajouter un sulfate de vanadyle dissous dans l'eau, sous atmosphère d'azote, à une solution du dérivé de cystéine obtenu en (a), dans un mélange diméthylformamide tampon borate à un pH d'environ 10, dans un rapport cystéine:vanadyle de 5:1, récupérer le complexe précité obtenu et laver à l'eau et sécher ledit complexe précité.

Un second procédé de préparation du composé ayant un groupement amine libre ou des groupements amine et carboxyle substitués est caractérisé en ce que :

- on fait réagir la cystéïne ou un dérivé de celle-ci avec du vanadyle sulfate à un pH d'environ 7 dans l'eau,
- on récupère le complexe obtenu après évaporation,
- on redissout ledit complexe dans du diméthylformamide,
- on procède au couplage avec une amine mono ou bifonctionnelle, un alcool mono ou bifonctionnel ou une amine-alcool, par l'intermédiaire de dimétnylaminopropyléthylcarbodiimide, et
- on récupère le complexe après évaporation sous vide du solvant, et on lave ledit complexe à l'éther et à l'eau.

L'invention a également pour objet une composition pharmaceutique, notamment antidiabétique, comprenant comme substance active le composé tel que défini précédemment.

Une telle composition présente une activité anti-diabétique et est non toxique, elle peut être administrée oralement, ce qui offre un avantage considérable pour les personnes souffrant de diabète insulino-dépendant.

L'invention sera mieux comprise, et d'autres buts, caractéristiques et détails de celle-ci apparaîtront plus clairement à la lecture de la description explicative qui va suivre, faite en référence aux exemples illustratifs et non limitatifs.

Le nouveau composé organo-minéral de vanadyle résulte de la complexation d'un vanadyle et d'une cystéïne ou dérivé de cystéïne et présente la formule générale (I).

Plus précisément, ce complexe levogyre (L) ou dextrogyre (D) présente un groupement amine libre ou un groupement carboxyle libre ou des groupements amine et carboxyle substitués.

a) Dans le cas où le ligand comprend un groupement amine libre, il peut être d'une part bimoléculaire et a alors pour formule générale :

$$VO^{2+}\left[NH_2 - \underset{\displaystyle CH_2S^-}{\underset{|}{CH}} - COX\right]_2 \qquad (II)$$

D'autre part, il peut être monomoléculaire et a donc pour formule générale :

$$VO^{2+}\left[NH_2 - \underset{\displaystyle CH_2S^-}{\underset{|}{CH}} - CO\right]_2 - X \qquad (III)$$

b) Dans le cas où le ligand comprend un groupement carboxyle libre, il peut de façon similaire, d'une part être bimoléculaire et a pour formule générale:

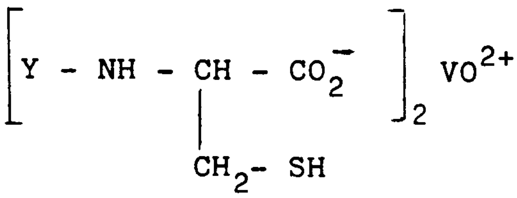

(IV)

Le ligand peut d'autre part être monomoléculaire et a pour formule générale :

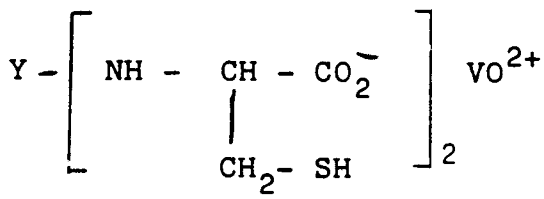

(V)

c) Dans le cas où le ligand comprend des groupements amine et carboxyle substitués, il se présente sous forme monomoléculaire et a la formule générale suivante:

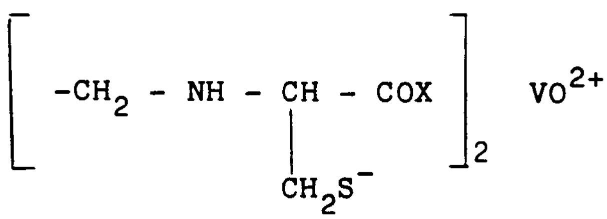

(VI)

Dans les formules (II) et (VI), X est un groupement -OR ou un groupement -NHR (amine), dans lequel le radical R est un groupement aryle, aralkyle ou alkyle autre que méthyle.

Ainsi, dans le cas où X est un groupement -NHR, le composé organo-minéral est par exemple un complexe de vanadyle avec une amide de la cystéïne telle que notamment une butylamide de la cystéïne ou une octylamide de la cystéïne. Le complexe (L) vanadyle - octylamide de la cystéïne est illustré par la formule suivante :

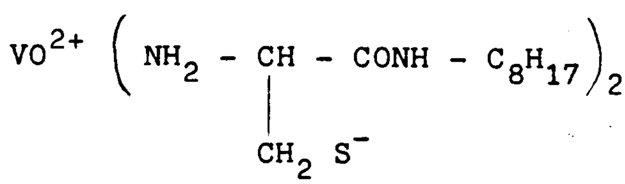

De plus, dans le cas où X est un groupement -OR, le composé organo-minéral est par exemple un complexe de vanadyle avec un ester de la cystéïne tel que notamment un octylester de la cystéïne ou un butylester de la cystéïne.

En outre, dans la formule (III), X correspond à l'un des groupements suivants :

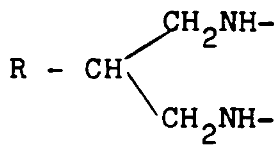

(amine bifonctionnelle)

ou

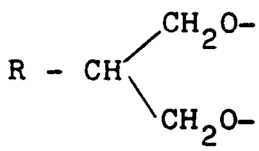

(alcool bifonctionnel)

ou 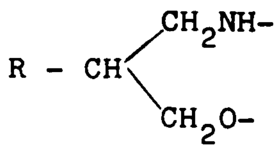

(amine-alcool)

dans lesquels R est un groupement aryle, aralkyle ou alkyle autre que méthyle.

Dans la formule (IV), Y correspond à un groupement R'-CO- (cétone) dans lequel le radical R' est un groupement alkyle, aryle ou aralkyle.

Dans la formule (V), Y correspond à l'un des groupements suivants :

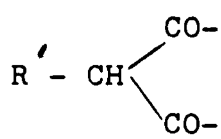

ou

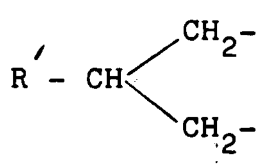

dans lesquels R' est également un groupement alkyle, aryle ou aralkyle.

A titre d'exemple, on donnera ci-après la préparation des composés, à savoir les complexes de vanadyle de formule : (II), (III), (IV), (V) et (VI).

(i) Un procédé de préparation du composé (II) ou (VI) consiste :

- à faire réagir une amine monofonctionnelle ou un alcool monofonctionnel avec la cystéïne ou dérivé de celle-ci protégée sur la fonction amine et la fonction thiol par un groupement BOC ou tertiobutyloxycarbonyl en présence de dicyclohexylcarbodimide/hydroxybenzotriazole,
- à éliminer ledit groupement BOC ou tertiobutyloxycarbonyl par acidolyse au moyen d'acide chlorhydrique et de Dioxanne,
- à ajouter du sulfate de vanadyle qui est dissous dans l'eau, sous atmosphère d'azote, à la solution de chlorhydrate de dérivé de cystéïne dans un mélange diméthylformamide-tampon borate à un pH d'environ 10 dans un rapport cystéïne/vanadyle de 5/1,
- puis, à agiter le mélange pendant 2 heures sous atmosphère d'azote,

- à récupérer le complexe formé par précipitation, au moyen d'une filtration, et
- à laver à l'eau le précipité et à le sécher.

Le produit obtenu présente les caractéristiques suivantes. Il est insoluble dans l'eau et dans l'éther éthylique. Il est par contre soluble dans le DMF ou diméthylformamide et dans le méthanol.

L'exemple 1 ci-dessous illustre un mode de réalisation de ce procédé dans le cas de la préparation d'un complexe de vanadyle et d'une octylamide de la cystéïne.

(ii) La préparation du composé de formule (III) consiste à faire réagir une amine bifonctionnelle ou un alcool bifonctionnel ou une amine-alcool avec la cystéïne ou dérivé de celle-ci. Les opérations suivantes sont identiques à celles du procédé de préparation décrit ci-dessus.

(iii) Un autre procédé de préparation du composé de formule (II) ou (VI), consiste :

- à faire réagir la cystéïne ou un dérivé de celle-ci avec du vanadyle sulfate à un pH d'environ 7 dans l'eau,
- à récupérer le complexe obtenu après évaporation,
- à redissoudre le complexe formé dans du dimethylformamide comme ci-dessus,
- à procéder au couplage avec une amine monofonctionnelle ou un alcool monofonctionnel par l'intermédiaire de diméthylaminopropyléthylcarbodiimide, et
- à récupérer le complexe après évaporation sous vide du solvant, lavage à l'éther et à l'eau, dissolution dans le méthanol, évaporation et reprécipitation par l'éther éthylique.

Cet autre procédé est illustré par l'exemple 2 ci-dessous dans le cas de la préparation d'un complexe de vanadyle avec l'octylamide de la cystéïne.

(iv) La préparation du composé (II) suit les mêmes étapes que le procédé mentionné ci-dessus pour la préparation des composés (II) et (VI) ; mais, comme produits de départ, on fait réagir une amine bifonctionnelle ou un alcool bifonctionnel ou une amine-alcool avec la cystéïne ou dérivé de celle-ci.

(v) Le procédé de préparation du composé de formule (IV) où Y correspond au groupement R-CO-, consiste:

- à coupler le dérivé activé (ester ou chlorure) de l'acide R-COOH avec la cystéïne préalablement protégée sur sa fonction thiol par un groupement BOC,
- à déprotéger ensuite la fonction thiol par acidolyse ; et
- à complexer le dérivé N-acylé obtenu avec le vanadyle en milieu DMF-eau à pH 10, un tel milieu contenant le sulfate de vanadyle.

(vi) Enfin, le procédé de préparation du composé de formule (V) où Y correspond à :

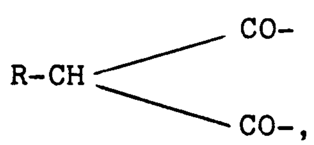

consiste :

- à coupler le dérivé activé (ester ou chlorure) du diacide

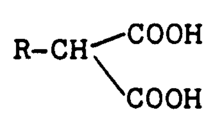

avec la cystéine préalablement protégée sur la fonction thiol par un groupement BOC,
- à déprotéger la fonction thiol par acidolyse,
- à réduire le dérivé N-acylé ainsi obtenu ; et
- à complexer ensuite ce dérivé N-acylé en milieu DMF - eau à pH 10, en présence de sulfate de vanadyle.

<u>Exemple 1.</u>

On fait réagir selon le procédé (i) ci-dessus, du sulfate de vanadyle dissous dans l'eau avec une solution de chlorhydrate de la cystéïne octylamide de formule suivante :

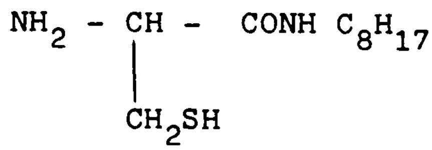

sous atmosphère d'azote dans un mélange diméthylformamide-tampon borate (pH de l'ordre de 10) dans un rapport molaire de la cystéïne au vanadyle de 5: 1. Le produit est obtenu par précipitation.

Au moyen d'un dosage par absorption atomique du vanadium, on obtient une stoechiométrie de 2:1 de la cystéïne par rapport au vanadyle et un pourcentage en poids de vanadium de l'ordre de 9,6% + 0,2%.

Le produit ainsi obtenu est un complexe ou ligand bimoléculaire.

En outre, l'analyse RPE ou résonance paramagnétique électronique en référence à la figure 1, présente des bandes de résonance de structure hyperfine. Il résulte de cette analyse que le vanadium est sous forme vanadyle $VO^{2+}$.

Par conséquent, le composé organo-minéral de vanadyle ainsi obtenu, a pour formule :

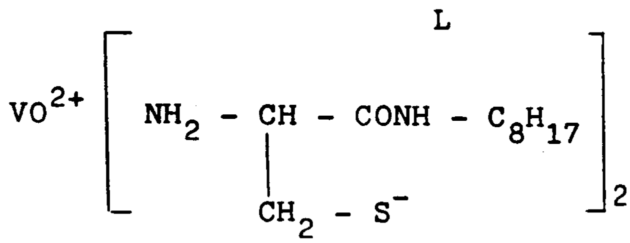

Exemple 2.

On couple le vanadyle sulfate avec l'octylamine de cystéïne selon le procédé (iii) mentionné ci-dessus.

Le composé obtenu est un complexe (L) de vanadyle et d'octylamide de la cystéïne de formule :

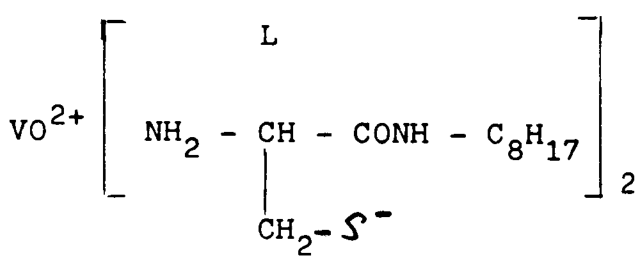

Le composé organo-minéral de vanadyle précédemment défini est utilisé notamment dans l'industrie pharmaceutique. Plus particulièrement, on a démontré qu'une composition pharmaceutique contenant comme substance active un tel complexe, a une activité anti-diabétique et est non toxique. Ainsi, de tels dérivés organo-minéraux du vanadyle décrits ci-dessus, sont des substituts thérapeutiques de l'insuline, administrables par voie orale dans les cas de diabètes insulino-dépendants.

Dans un premier temps, on a étudié la toxicité potentielle du complexe.

On a réalisé l'étude à partir du composé de vanadyle bimoléculaire avec un groupement amine libre, de formule générale (II). Et on a comparé la toxicité relevée après administration unique par voie orale chez le rat, d'une part du composé de formule (II) et d'autre part, du sulfate de vanadyle.

Le protocole des essais toxicologiques est le suivant.

D'une part, on recherche la toxicité du composé (II) en administrant oralement des doses croissantes du composé (II), allant jusqu'à 1g/kg, à 1 lot de 10 rats mâles de souche WISTAR et d'un poids moyen de 150g. On effectue l'administration chez des animaux à jeùn, par gavage, à une quantité de 10 ml/kg d'une suspension du composé (II) dans la gomme arabique à 3%. Puis, on suit les animaux pendant 14 jours, afin de comptabiliser les mortalités et de rechercher et observer les signes de toxicité.

D'autre part, on recherche la toxicité aigüe du sulfate de vanadyle ($VOSO_4$) dans des conditions similaires sur des animaux répartis en 7 lots de 10 animaux auxquels on a administré les doses suivantes : 300, 400, 500, 600, 800, 1000 et 1000 mg/kg.

On a calculé la dose léthale ($DL_{50}$) par la méthode de LITCHFIELD et WILCOXON.

Les résultats sont les suivants.

Sur les rats ayant absorbé le composé (II), on n'observe aucun signe de toxicité apparent jusqu'à la dose de 1g/kg. La dose de 1g/kg correspond environ à 20 fois la dose active anti-diabétique.

Sur les rats ayant absorbé le sulfate de vanadyle, la $DL_{50}$ s'établit à 600 mg/kg. On observe les premiers signes de toxicité dès la dose de 400 mg/kg. La toxicité se manifeste par une diarrhée de sévérité variable, une diminution d'activité motrice et un amaigrissement. Pour les doses les plus élevées : 800, 1000 et 1500 mg/kg, on observe une hypothermie et des cas de chromodacryorrhée.

Le composé organo-minéral de vanadyle est donc non toxique comparé au sulfate de vanadyle, pour des administrations du composé allant jusqu'à 1g par kg.

Dans un second temps, on a étudié l'activité anti-diabétique du complexe de vanadyle et de cystéïne décrit ci-dessus.

On a réalisé également l'étude à partir du composé de formule (II). Et, on a comparé l'activité de ce composé entre des rats diabétiques et des rats non diabétiques et l'activité de ce composé (II) avec celle du sulfate de vanadyle.

On a mis en place des essais sur des animaux ayant une glycémie supérieure à 3g/l.

La méthode est la suivante. On induit le diabète par injection intraveineuse d'une dose unique de streptozotocine de l'ordre de 50 mg/kg, chez des rats mâle ou femelle de la race WISTAR d'un poids moyen de 200g. Et on dose la glycémie 4 jours après l'injection.

Le protocole des essais pharmacologiques se déroule comme suit. On administre le composé (II) à une quantité de 45 mg/kg, par gavage, chez des animaux non à jeùn, sous la forme d'une suspension à raison de 10 ml/kg dans une solution de gomme arabique à 3%. Et on administre une dose de sulfate de vanadyle équimolaire à 45 mg/kg du composé (II).

On mesure la glycémie par dosages. On effectue les prélèvements sanguins à la queue de l'animal. On dose la glycémie au moyen d'un dispositif dénommé "glucometer AMES", (marque déposée). On lit le taux de glycémie sur des bandelettes réactives "Derotrostix", (marque déposée), du dispositif précité.

Par ailleurs, on dose l'insulinémie par kit radio-immunologique dénommé "AMERSHAM", (marque déposée).

Les résultats sont les suivants.

En référence à la figure 2, on observe l'effet d'une administration unique du composé (II) à la dose de 45 mg/kg sur la glycémie et sur l'insulinémie d'animaux diabétiques ou non diabétiques.

Sur la figure 2,

—— ○ —— correspond à l'effet de l'administration orale de 45 mg/kg du composé (II) sur la glycémie d'animaux non diabétiques ;

—— ● —— correspond à l'effet de l'administration orale de 45 mg/kg du composé (II) sur la glycémie d'animaux diabétiques ;

------□------ correspond à l'effet de l'administration orale de 45 mg/kg du composé (II) sur l'insulinémie d'animaux non diabétiques ;

-----■----- correspond à l'effet de l'administration orale de 45 mg/kg du composé (II) sur l'insulinémie d'animaux diabétiques.

Chez l'animal diabétique, l'administration du composé de formule (II) induit une chute de la glycémie qui dure environ 24 heures. L'effet maximum se situe entre la cinquième et la septième heure après l'administration. Au-delà de 7 heures, la glycémie diminue progressivement pour retrouver un taux normal tel que celui des animaux non diabétiques.

Il est à noter ici que la glycémie des animaux diabétiques ou non diabétiques non traités ne varie pas plus de 0,2 g/l autour de la valeur de base pendant la période expérimentale considérée.

De plus, il est également à noter que l'administration d'une dose de sulfate de vanadyle équimolaire à 45mg/kg du composé (II) induit une diminution plus faible et transitoire de la glycémie des animaux diabétiques. En effet, la diminution maximale est de l'ordre de 1g/l et la durée de l'effet se limite à 2 heures après l'administration.

Par conséquent, le composé organo-minéral de vanadyle, administré par voie orale, à une dose non toxique, présente des propriétés hypoglycémiantes uniquement chez l'animal diabétique. L'action de ce composé est prolongée dans le temps et est supérieure à celle du sulfate de vanadyle.

Pour l'insulinémie, la figure 2 montre qu'elle diminue ou reste stable chez des animaux diabétiques ou non.

De ce fait, l'administration du composé (II) n'entraîne pas la fabrication d'insuline. Il n'y a pas d'hyperinsulinémie. Au contraire, on observe une chute de l'insulinémie. Dans ces conditions, le composé organo-minéral de vanadyle décrit précédemment, se substitue à l'insuline, ce qui présente un avantage surprenant. En effet, l'action anti-diabétique du composé ne s'accompagne pas d'une augmentation de l'insulinémie.

En vue de ce qui précède, la complexation de l'ion vanadyle par un dérivé de la cystéine permet d'obtenir une activité anti-diabétique supérieure et une toxicité inférieure à celle du sulfate de vanadyle, et ne s'accompagnant pas d'hyperinsulinémie.

Des compositions pharmaceutiques sont ainsi préparées à base du complexe décrit ci-dessus en tant que substance active. Ces compositions pharmaceutiques comprennent outre le dérivé organo-minéral de vanadyle, des excipients.

Elles se présentent soit sous une forme solide telle que capsules, dragées ou analogues, soit sous une forme soluble telle que suspension ou gouttes ou analogues.

Les personnes souffrant de diabètes insulino-dépendants peuvent alors être soignées par voie orale, et ainsi être libérées de toute astreinte liée à un traitement par voie parentérale, sans rencontrer de risque de toxicité.

## Revendications

**Revendications pour les Etats contractants suivants : AT, BE, CH, DE, GB, IT, LI, LU, NL, SE**

**1.** Composé organo-minéral de vanadyle, caractérisé en ce qu'il se présente sous la forme d'un complexe de vanadyle et de cystéine ayant la formule générale suivante :

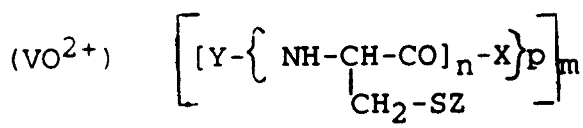

(I)

dans laquelle n, p et m sont respectivement des nombres entiers ayant pour valeur 1 ou 2 et

* dans le cas où p = 1 :

- Y représente un atome d'hydrogène, un groupe $-CH_2-$ ou un groupe R'-CO, dans lequel R' est un groupe alkyle, aryle ou aralkyle ;
- Z représente une charge - ou un atome d'hydrogène et

(a) lorsque n = 1 et m = 2, X représente un groupe $O^-$, un groupe OR ou un groupe NHR, R étant un groupe aryle, aralkyle ou alkyle, à l'exclusion du groupe méthyle, à condition que :
lorsque X représente un groupe $O^-$,
Z représente un atome d'hydrogène et
Y représente un groupe R'-CO
et
lorsque X représente un groupe OR ou un groupe NHR,
Z représente une charge - et
Y représente un groupe $-CH_2-$ ou un atome d'hydrogène
et
(b) lorsque n = 2 et m = 1, X représente l'un des groupes suivants :

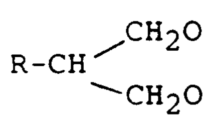

,

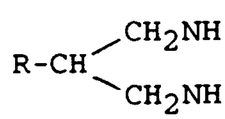

ou

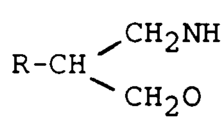

R étant un groupe aryle ou aralkyle ou un groupe alkyle, à l'exclusion du groupe méthyle et
Z représente une charge et
Y représente un atome d'hydrogène et
* dans le cas où p = 2

- n = 1
- m = 1
- X représente un groupe $O^-$,
- Z représente un atome d'hydrogène et
- Y représente un groupe

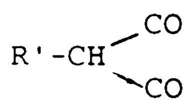

ou
un groupe

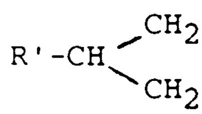

,

R' étant un groupe alkyle, aryle ou aralkyle.

**2.** Composé selon la revendication 1, caractérisé en ce que la cystéine est sous sa forme L.

**3.** Composé selon la revendication 1, caractérisé en ce que la cystéine est sous sa forme D.

**4.** Composé selon la revendication 1, caractérisé en ce que p = 1, n = 1, m = 2, X représente un groupe OR ou un groupe NHR dans lesquels R est un groupe aryle, aralkyle ou alkyle, à l'exclusion du groupe méthyle, lequel composé correspond à l'une quelconque des formules II ou VI :

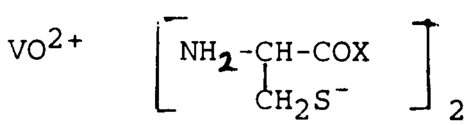

(II)

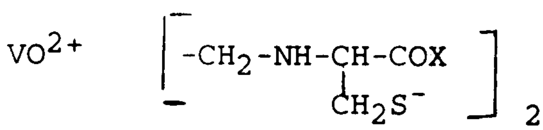

(VI)

**5.** Composé selon la revendication 1, caractérisé en ce que p = 1, n = 1, m = 2, Y représente un groupe R'-CO dans lequel R' est un groupe alkyle, aryle ou aralkyle, lequel composé correspond à la formule IV suivante :

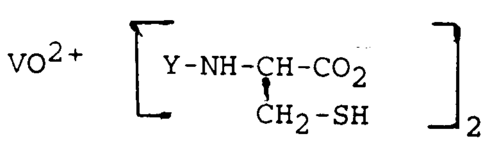

(IV)

**6.** Composé selon la revendication 1, caractérisé en ce que p = 1, n = 2, m = 1, et X représente l'un des groupes suivants :

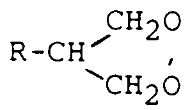 , 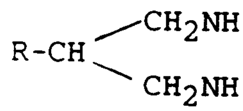 ou 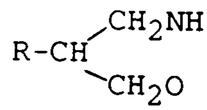

dans lesquels
R est un groupe aryle, aralkyle ou alkyle, à l'exclusion du groupe méthyle,
lequel composé correspond à la formule III suivante :

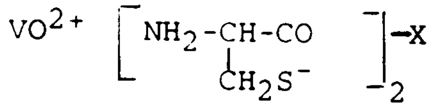

(III)

7. Composé selon la revendication 1, caractérisé en ce que p = 2, n = 1, m = 1, et Y représente un groupe

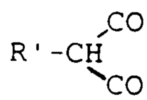

ou un groupe

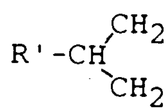 ,

dans lesquels
R' est un groupe alkyle, aryle ou aralkyle, lequel composé correspond à la formule V suivante :

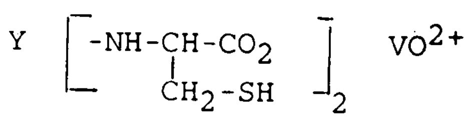

(V)

8. Procédé de préparation du composé de formule I selon la revendication 1, caractérisé en ce qu'il consiste à :
(a) préparer, au cours d'une première étape, un dérivé de cystéine, en faisant réagir une amine monoou bifonctionnelle ou un alcool mono- ou bifonctionnel, ou une amine-alcool avec la cystéine ou un dérivé de celle-ci, protégé sur la fonction amine et la fonction thiol par un groupement tertiobutyloxycarbonyl en présence de dicyclohexyl carbodiimide/hydroxybenzotriazole, et en éliminant ledit groupement tertiobutyloxycarbonyl par acidolyse et dans une seconde étape
(b) ajouter un sulfate de vanadyle dissous dans l'eau, sous atmosphère d'azote, à une solution du dérivé de cystéine obtenu en (a), dans un mélange diméthylformamide tampon borate à un pH d'environ 10, dans un rapport cystéine:vanadyle de 5:1, récupérer le complexe précité obtenu et laver à l'eau et sécher ledit complexe précité.

9. Méthode de préparation du composé de formule I, selon la revendication 1, caractérisé en ce qu'il consiste à :
- faire réagir la cystéine ou un dérivé de celle-ci avec un sulfate de vanadyle à un pH d'environ 7 dans l'eau,
- récupérer le complexe obtenu après évaporation,
- redissoudre ledit complexe dans du diméthylformamide,

- procéder au couplage avec une amine mono ou bifonctionnelle, un alcool mono ou bifonctionnel ou une amine-alcool, par l'intermédiaire du diméthylaminopropyléthylcarbodiimide, et
- récupérer le complexe après évaporation sous vide du solvant, et
- laver ledit complexe à l'éther et à l'eau.

**10.** Composition pharmaceutique, notamment antidiabétique, caractérisée en ce qu'elle comprend comme substance active le composé tel que défini selon l'une des revendications 1 à 7.

**11.** Composition pharmaceutique selon la revendication 10, caractérisée en ce qu'elle se présente sous une forme solide telle que capsules, dragées ou analogues.

**12.** Composition pharmaceutique selon la revendication 10, caractérisée en ce qu'elle se présente sous une forme soluble telle que suspension ou gouttes ou analogues.

**13.** Composition pharmaceutique selon l'une des revendications 10 à 12, caractérisée en ce qu'elle est utile pour l'obtention d'un médicament antidiabétique destiné à être administré oralement.

**Revendications pour les Etats contractants suivants : ES, GR**

**1.** Procédé de préparation d'un composé organominéral de vanadyle se présentant sous la forme d'un complexe de vanadyle et de cystéine ayant la formule générale suivante :

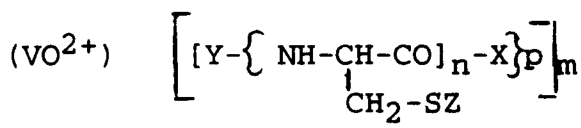
(I)

dans laquelle n, p et m sont respectivement des nombres entiers ayant pour valeur 1 ou 2 et

* dans le cas où p = 1 :

- Y représente un atome d'hydrogène, un groupe $-CH_2-$ ou un groupe R'-CO, dans lequel R' est un groupe alkyle, aryle ou aralkyle ;
- Z représente une charge - ou un atome d'hydrogène et
  (a) lorsque n = 1 et m = 2, X représente un groupe $O^-$, un groupe OR ou un groupe NHR, R étant un groupe aryle, aralkyle ou alkyle, à l'exclusion du groupe méthyle,
  à condition que :
  lorsque X représente un groupe $O^-$,
  Z représente un atome d'hydrogène et
  Y représente un groupe R'-CO
  et
  lorsque X représente un groupe OR ou un groupe NHR,
  Z représente une charge - et
  Y représente un groupe $-CH_2-$ ou un atome d'hydrogène
  et
  (b) lorsque n = 2 et m = 1, X représente l'un des groupes suivants :

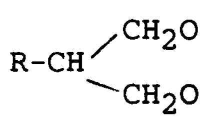
,
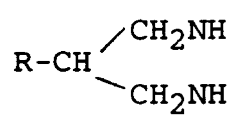

ou
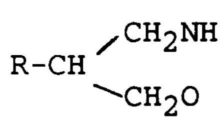

R étant un groupe aryle ou aralkyle ou un groupe alkyle, à l'exclusion du groupe méthyle et

Z représente une charge et
Y représente un atome d'hydrogène et
* dans le cas où p = 2

- n = 1
- m = 1
- X représente un groupe $O^-$,
- Z représente un atome d'hydrogène et
- Y représente
  un groupe

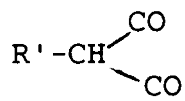

ou
un groupe

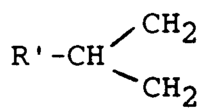

,

R' étant un groupe alkyle, aryle ou aralkyle, caractérisé en ce qu'il consiste à :
(a) préparer, au cours d'une première étape, un dérivé de cystéine, en faisant réagir une amine monoou bifonctionnelle ou un alcool mono- ou bifonctionnel, ou une amine-alcool avec la cystéine ou un dérivé de celle-ci, protégé sur la fonction amine et la fonction thiol par un groupement tertiobutyloxycarbonyl en présence de dicyclohexyl carbodiimide/hydroxybenzotriazole, et en éliminant ledit groupement tertiobutyloxycarbonyl par acidolyse et dans une seconde étape
(b) ajouter un sulfate de vanadyle dissous dans l'eau, sous atmosphère d'azote, à une solution du dérivé de cystéine obtenu en (a), dans un mélange diméthylformamide tampon borate à un pH d'environ 10, dans un rapport cystéine:vanadyle de 5:1, récupérer le complexe précité obtenu et laver à l'eau et sécher ledit complexe précité.

**2.** Procédé de préparation d'un composé organominéral de vanadyle se présentant sous la forme d'un complexe de vanadyle et de cystéine ayant la formule I selon la revendication 1, caractérisé en ce qu'il consiste à :
   - faire réagir la cystéine ou un dérivé de celle-ci avec un sulfate de vanadyle à un pH d'environ 7 dans l'eau,
   - récupérer le complexe obtenu après évaporation,
   - redissoudre ledit complexe dans du diméthylformamide,
   - procéder au couplage avec une amine mono ou bifonctionnelle, un alcool mono ou bifonctionnel ou une amine-alcool, par l'intermédiaire du diméthylaminopropyléthylcarbodiimide, et
   - récupérer le complexe après évaporation sous vide du solvant, et
   - laver ledit complexe à l'éther et à l'eau.

**3.** Procédé de préparation selon la revendication 1 ou la revendication 2, caractérisé en ce que la cystéine est sous sa forme L.

**4.** Procédé de préparation selon la revendication 1 ou la revendication 2, caractérisé en ce que la cystéine est sous sa forme D.

**5.** Procédé de préparation selon l'une quelconque des revendications 1 à 4, caractérisé en ce que l'on obtient un composé de formule L dans laquelle p = 1, n = 1, m = 2, X représente un groupe OR ou un groupe NHR dans lesquels R est un groupe aryle, aralkyle ou alkyle, à l'exclusion du groupe méthyle, lequel composé correspond à l'une quelconque des formules II ou VI :

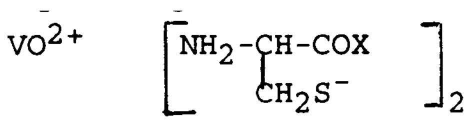

(II)

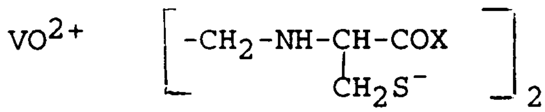

(VI)

6. Procédé de préparation selon l'une quelconque des revendications 1 à 4, caractérisé en ce que l'on obtient un composé de formule I dans laquelle p = 1, n = 1, m = 2, Y représente un groupe R'-CO dans lequel R' est un groupe alkyle, aryle ou aralkyle, lequel composé correspond à la formule IV suivante :

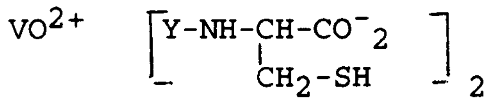

(IV)

7. Procédé de préparation selon l'une quelconque des revendications 1 à 4, caractérisé en ce que l'on obtient un composé de formule I dans laquelle p = 1, n = 2, m = 1, et X représente l'un des groupes suivants :

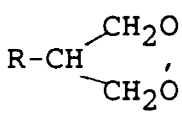 , 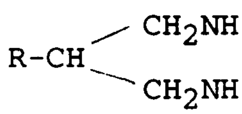 ou 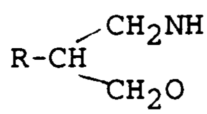

dans lesquels
R est un groupe aryle, aralkyle ou alkyle, à l'exclusion du groupe méthyle,
lequel composé correspond à la formule III suivante :

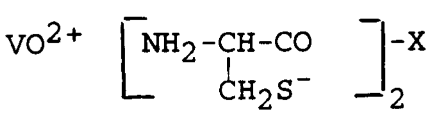

(III)

8. Procédé de préparation selon l'une quelconque des revendications 1 à 4, caractérisé en ce que l'on obtient un composé de formule I dans laquelle p = 2, n = 1, m = 1, et Y représente
un groupe

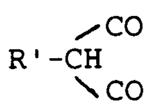

ou un groupe

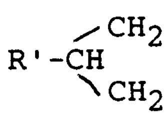 ,

dans lesquels
R' est un groupe alkyle, aryle ou aralkyle,
lequel composé correspond à la formule V suivante :

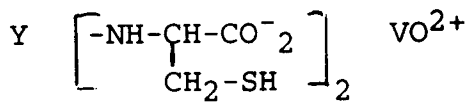

(V)

9. Procédé de préparation d'une composition pharmaceutique, caractérisé en ce que l'on introduit dans ladite composition, un composé obtenu selon l'une quelconque des revendications 1 à 8.

10. Procédé de préparation d'une composition selon la revendication 9, caractérisé en ce qu'on formule ladite composition sous une forme solide telle que capsules, dragées ou analogues.

11. Procédé de préparation d'une composition selon la revendication 9, caractérisé en ce qu'on formule ladite composition sous une forme soluble telle que suspension ou gouttes ou analogues.

12. Application d'une composition obtenue selon l'une quelconque des revendications 9 à 11 à la préparation d'un médicament antidiabétique destiné à être administré oralement.

**Claims**
**Claims for the following Contracting States : AT, BE, CH, DE, GB, IT, LI, LU, NL, SE**

1. An organic-inorganic vanadyl compound, characterized in that it is in the form of a complex of vanadyl and cysteine having the following general formula:

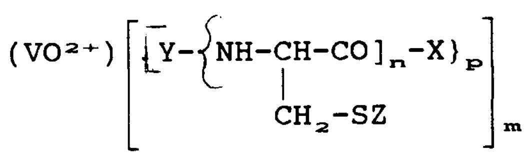

(I)

in which n, p and m are respectively integers having a value of 1 or 2 and
* in the case where p = 1:
- Y is a hydrogen atom, a group $-CH_2-$ or a group R'-CO, in which R' is an alkyl, aryl or aralkyl group;
- Z is a - charge or a hydrogen atom; and
  (a) when n = 1 and m = 2, X is a group $O^-$, a group OR or a group NHR, R being an aryl, aralkyl or alkyl group, with the exclusion of the methyl group, with the proviso that:
  when X is a group $O^-$,
  Z is a hydrogen atom and
  Y is a group R'-CO,
  and
  when X is a group OR or a group NHR,
  Z is a - charge and
  Y is a group $-CH_2-$ or a hydrogen atom,
  and
  (b) when n = 2 and m = 1, X is one of the following groups:

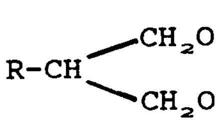 , 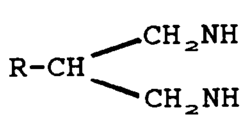 or 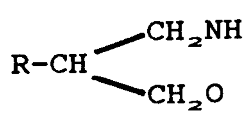 ,

R being an aryl or aralkyl group or an alkyl group, with the exclusion of the methyl group,
Z is a - charge and
Y is a hydrogen atom; and
* in the case where p = 2:

- n = 1,
- m = 1,
- X is a group $O^-$,
- Z is a hydrogen atom and
- Y is a group

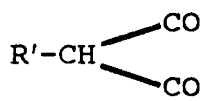

or a group

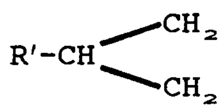

,

R' being an alkyl, aryl or aralkyl group.

2. A compound according to Claim 1, characterized in that the cysteine is in its L form.

3. A compound according to Claim 1, characterized in that the cysteine is in its D form.

4. A compound according to Claim 1, characterized in that p = 1, n = 1, m = 2 and X is a group OR or a group NHR, in which R is an aryl, aralkyl or alkyl group, with the exclusion of the methyl group, which compound has either one of formulae II and VI:

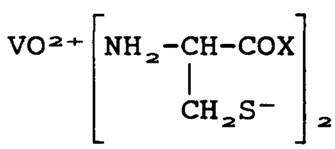

(II)

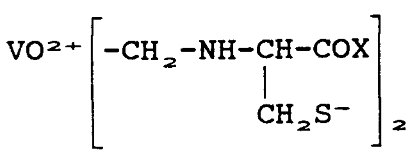

(VI)

5. A compound according to Claim 1, characterized in that p = 1, n = 1, m = 2 and Y is a group R'-CO, in which R' is an alkyl, aryl or aralkyl group, which compound has formula IV below:

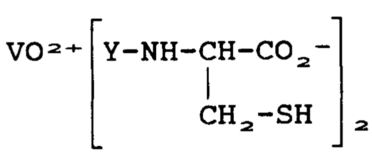

(IV)

**6.** A compound according to Claim 1, characterized in that p = 1, n = 2, m = 1 and X is one of the following groups:

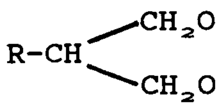 , 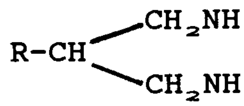 or 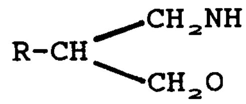 ,

in which R is an aryl, aralkyl or alkyl group, with the exclusion of the methyl group, which compound has formula III below:

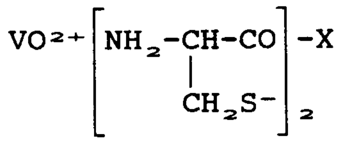 (III)

**7.** A compound according to claim 1, characterized in that p = 2, n = 1, m = 1 and Y is a group

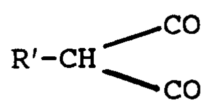

or a group

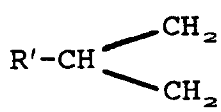 ,

in which R' is an alkyl, aryl or aralkyl group, which compound has formula V below:

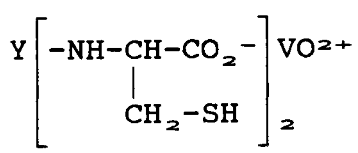 (V)

**8.** A method of preparing the compound of formula I according to Claim 1, characterized in that it consists:

(a) in a first step, in preparing a cysteine derivative by reacting a monofunctional or bifunctional amine, a monofunctional or bifunctional alcohol or an amine-alcohol with cysteine or a cysteine derivative protected on the amine group and the thiol group by a tert-butoxycarbonyl group, in the presence of dicyclohexylcarbodiimide/hydroxybenzotriazole, and by removing said tert-butoxycarbonyl group by acidolysis, and

(b) in a second step, in adding an aqueous solution of a vanadyl sulphate, under a nitrogen atmosphere, to a solution of the cysteine derivative obtained in (a) in a dimethylformamide/borate buffer mixture at a pH of about 10, in a cysteine:vanadyl ratio of 5:1, recovering the above-mentioned complex obtained, washing said above-mentioned complex with water and drying it.

9. A method of preparing the compound of formula I according to Claim 1, characterized in that it consists in:
   - reacting cysteine or a cysteine derivative with a vanadyl sulphate at a pH of about 7 in water,
   - recovering the complex obtained after evaporation,
   - redissolving said complex in dimethylformamide,
   - carrying out a coupling reaction with a monofunctional or bifunctional amine, a monofunctional or bifunctional alcohol or an amine-alcohol, in the presence of dimethylaminopropylethylcarbodiimide,
   - recovering the complex after evaporation of the solvent under vacuum, and
   - washing said complex with ether and water.

10. A pharmaceutical composition, especially an antidiabetic composition, characterized in that it comprises the compound such as defined according to one of Claims 1 to 7 as the active substance.

11. A pharmaceutical composition according to Claim 10, characterized in that it is in a solid form such as capsules, coated tablets or the like.

12. A pharmaceutical composition according to Claim 10, characterized in that it is in a soluble form such as a suspension, drops or the like.

13. A pharmaceutical composition according to one of Claims 10 to 12, characterized in that it is useful for obtaining an antidiabetic drug intended for oral administration.

**Claims for the following Contracting States : ES, GR**

1. A method of preparing an organic-inorganic vanadyl compound which is in the form of a complex of vanadyl and cysteine having the following general formula:

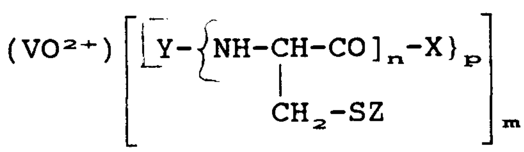 (I)

in which n, p and m are respectively integers having a value of 1 or 2 and
* in the case where P = 1:
   - Y is a hydrogen atom, a group $-CH_2-$ or a group R'-CO, in which R' is an alkyl, aryl or aralkyl group;
   - Z is a - charge or a hydrogen atom; and
     (a) when n = 1 and m = 2, X is a group $O^-$, a group OR or a group NHR, R being an aryl, aralkyl or alkyl group, with the exclusion of the methyl group, with the proviso that:
     when X is a group $O^-$,
     Z is a hydrogen atom and
     Y is a group R'-CO,
     and
     when X is a group OR or a group NHR,
     Z is a - charge and
     Y is a group $-CH_2-$ or a hydrogen atom,
     and
     (b) when n = 2 and m = 1, X is one of the following groups:

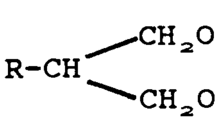 , 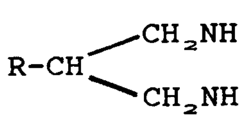 or 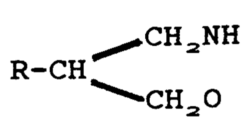 ,

R being an aryl or aralkyl group or an alkyl group, with the exclusion of the methyl group,
Z is a - charge and
Y is a hydrogen atom; and
* in the case where p = 2:

- n = 1,
- m = 1,
- X is a group $O^-$,
- Z is a hydrogen atom and
- Y is a group

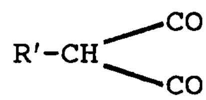

or a group

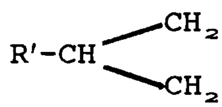
,

R' being an alkyl, aryl or aralkyl group, characterized in that it consists:

(a) in a first step, in preparing a cysteine derivative by reacting a monofunctional or bifunctional amine, a monofunctional or bifunctional alcohol or an amine-alcohol with cysteine or a cysteine derivative protected on the amine group and the thiol group by a tert-butoxycarbonyl group, in the presence of dicyclohexylcarbodiimide/hydroxybenzotriazole, and by removing said tert-butoxycarbonyl group by acidolysis, and

(b) in a second step, in adding an aqueous solution of a vanadyl sulphate, under a nitrogen atmosphere, to a solution of the cysteine derivative obtained in (a) in a dimethylformamide/borate buffer mixture at a pH of about 10, in a cysteine:vanadyl ratio of 5:1, recovering the above-mentioned complex obtained, washing said above-mentioned complex with water and drying it.

**2.** A method of preparing an organic-inorganic vanadyl compound which is in the form of a complex of vanadyl and cysteine having the following general formula:

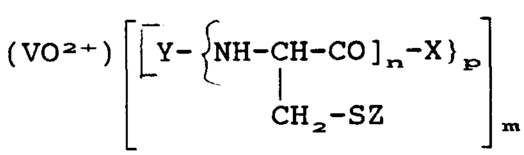
(I)

in which n, p and m are respectively integers having a value of 1 or 2 and
* in the case where p = 1:

- Y is a hydrogen atom, a group $-CH_2-$ or a group R'-CO, in which R' is an alkyl, aryl or aralkyl group;
- Z is a - charge or a hydrogen atom; and

(a) when n = 1 and m = 2, X is a group $O^-$, a group OR or a group NHR, R being an aryl, aralkyl or alkyl group, with the exclusion of the methyl group, with the proviso that:
when X is a group $O^-$,
Z is a hydrogen atom and
Y is a group R'-CO,
and
when X is a group OR or a group NHR,
Z is a - charge and
Y is a group $-CH_2-$ or a hydrogen atom,

and
(b) when n = 2 and m = 1, X is one of the following groups:

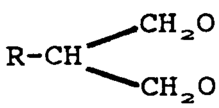 , 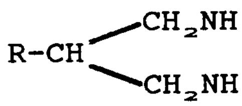 or 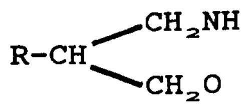 ,

R being an aryl or aralkyl group or an alkyl group, with the exclusion of the methyl group,
Z is a - charge and
Y is a hydrogen atom; and
* in the case where p = 2:
- n = 1,
- m = 1,
- X is a group $O^-$,
- Z is a hydrogen atom and
- Y is a group

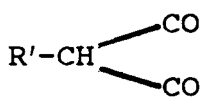

or a group

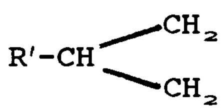 ,

R' being an alkyl, aryl or aralkyl group, characterized in that it consists in:
- reacting cysteine or a cysteine derivative with a vanadyl sulphate at a pH of about 7 in water,
- recovering the complex obtained after evaporation,
- redissolving said complex in dimethylformamide,
- carrying out a coupling reaction with a monofunctional or bifunctional amine, a monofunctional or bifunctional alcohol or an amine-alcohol, in the presence of dimethylaminopropylethylcarbodiimide,
- recovering the complex after evaporation of the solvent under vacuum, and
- washing said complex with ether and water.

**3.** A preparative method according to Claim 1 or Claim 2, characterized in that the cysteine is in its L form.

**4.** A preparative method according to Claim 1 or Claim 2, characterized in that the cysteine is in its D form.

**5.** A preparative method according to any one of Claims 1 to 4, characterized in that a compound of formula I is obtained in which p = 1, n = 1, m = 2 and X is a group OR or a group NHR, in which R is an aryl, aralkyl or alkyl group, with the exclusion of the methyl group, which compound has either one of formulae II and VI:

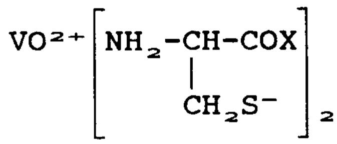

(II)

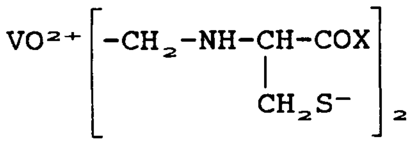

(VI)

**6.** A preparative method according to any one of Claims 1 to 4, characterized in that a compound of formula I is obtained in which p = 1, n = 1, m = 2 and Y is a group R'-CO, in which R' is an alkyl, aryl or aralkyl group, which compound has formula IV below:

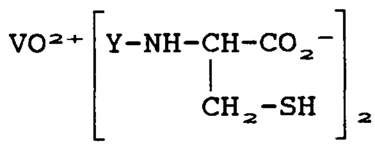

(IV)

**7.** A preparative method according to any one of Claims 1 to 4, characterized in that a compound of formula I is obtained in which p = 1, n = 2, m = 1 and X is one of the following groups:

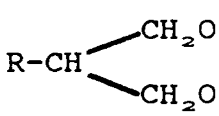 , 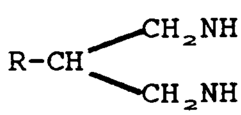 or 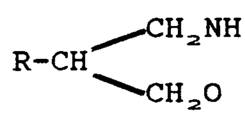 ,

in which R is an aryl, aralkyl or alkyl group, with the exclusion of the methyl group, which compound has formula III below:

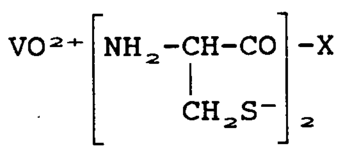

(III)

**8.** A preparative method according to any one of Claims 1 to 4, characterized in that a compound of formula I is obtained in which p = 2, n = 1, m = 1 and Y is a group

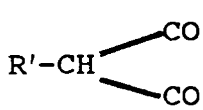

or a group

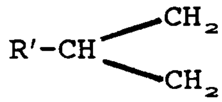,

in which R' is an alkyl, aryl or aralkyl group, which compound has formula V below:

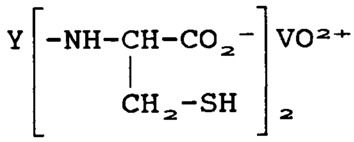 (V)

**9.** A method of preparing a pharmaceutical composition, characterized in that a compound obtained according to any one of Claims 1 to 8 is introduced into said composition.

**10.** A method of preparing a composition according to Claim 9, characterized in that said composition is formulated in a solid form such as capsules, coated tablets or the like.

**11.** A method of preparing a composition according to Claim 9, characterized in that said composition is formulated in a soluble form such as a suspension, drops or the like.

**12.** Application of a composition obtained according to any one of Claims 9 to 11 to the preparation of an antidiabetic drug intended for oral administration.

**Patentansprüche**

**Patentansprüche für folgende Vertragsstaaten : AT, BE, CH, DE, GB, IT, LI, LU, NL, SE**

**1.** Organisch-anorganische Vanadylverbindung, dadurch **gekennzeichnet,** daß sie in Form eines Komplexes aus Vanadyl und Cystein der folgenden allgemeinen Formel

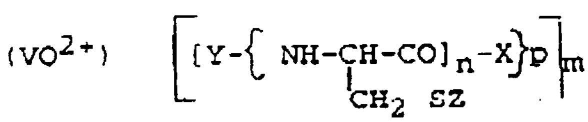 (I)

vorliegt, worin n, p und m jeweils ganze Zahlen mit dem Wert 1 oder 2 bedeuten, und

* in dem Falle, in dem p = 1 ist, Y für ein Wasserstoffatom, eine $-CH_2-$ oder eine R'-CO-Gruppe steht, worin R' eine Alkyl-, Aryl- oder Aralkylgruppe ist, Z eine negative Ladung oder ein Wasserstoffatom bedeutet, und

(a) wenn n = 1 und m = 2 sind, X für eine $O^-$-Gruppe, eine OR-Gruppe oder eine NHR-Gruppe steht, wobei R eine Aryl-, Aralkyl- oder Alkylgruppe mit Ausschluß der Methylgruppe bedeutet, mit der Maßgabe, daß, wenn X für eine $O^-$-Gruppe steht, Z für ein Wasserstoffatom steht und Y für eine R'-CO-Gruppe steht und wenn X für eine OR oder NHR-Gruppe steht, Z für eine negative Ladung steht und Y für cinc $-CH_2$-Gruppe oder ein Wasserstoffatom steht, und

(b) wenn n = 2 und m = 1 sind, x für eine der nachstehenden Gruppen

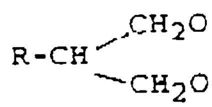 , 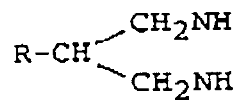

oder 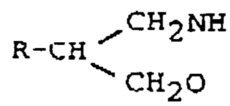

steht, wobei R eine Aryl- oder Aralkylgruppe oder eine Alkylgruppe, ausgenommen eine Methylgruppe, bedeutet, und Z eine Ladung bedeutet und Y für ein Wasserstoffatom steht, und

* im Falle, in dem p = 2 ist, n = 1, m = 1, X für eine $O^-$-Gruppe steht, Z für ein Wasserstoffatom steht und Y für eine Gruppe

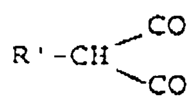

oder eine Gruppe

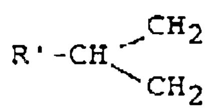

steht, wobei R' eine Alkyl-, Aryl- oder Aralkylgruppe bedeutet.

**2.** Verbindung nach Anspruch 1, dadurch **gekennzeichnet,** daß das Cystein in seiner L-Form vorliegt.

**3.** Verbindung nach Anspruch 1, dadurch **gekennzeichnet,** daß das Cystein in seiner D-Form vorliegt.

**4.** Verbindung nach Anspruch 1, dadurch **gekennzeichnet,** daß p = 1, n = 1, m = 2, X für eine OR-Gruppe oder eine NHR-Gruppe steht, worin R eine Aryl-, Aralkyl- oder Alkylgruppe, ausgenommen die Methylgruppe, bedeutet, wobei die Verbindung einer der Formeln II oder VI

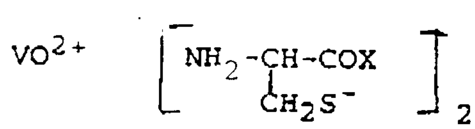 (II)

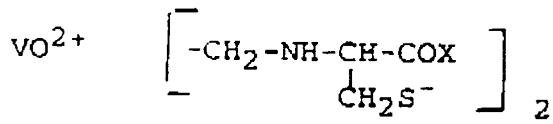 (VI)

entspricht.

**5.** Verbindung nach Anspruch 1, dadurch **gekennzeichnet,** daß p = 1, n = 1, m = 2, Y für eine R'-CO-Gruppe steht, worin R' eine Alkyl-, Aryl- oder Aralkylgruppe ist, wobei die Verbindung der folgenden Formel IV entspricht.

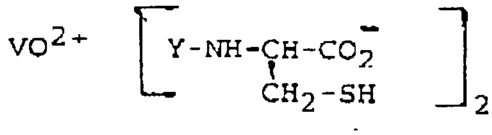

(IV)

6. Verbindung nach Anspruch 1, dadurch **gekennzeichnet,** daß p = 1, n = 2, m = 1 und X für eine der folgenden Gruppen

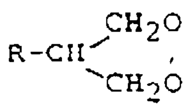

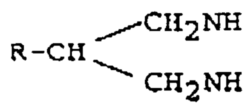

oder

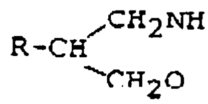

steht, worin R eine Aryl-, Aralkyl- oder Alkylgruppe bedeutet, ausgenommen die Methylgruppe, wobei die Verbindung der folgenden Formel III entspricht

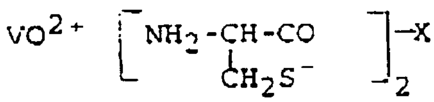

(III)

7. Verbindung nach Anspruch 1, dadurch **gekennzeichnet** daß p = 2, n = 1, m = 1 und Y für eine Gruppe

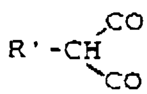

oder eine Gruppe

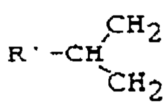

steht, worin R eine Alkyl-, Aryl- oder Aralkylgruppe bedeutet, wobei die Verbindung der folgenden Formel V

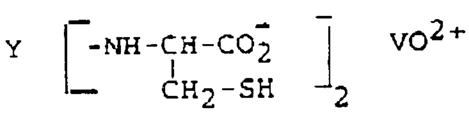

(V)

entspricht.

**8.** Verfahren zur Herstellung einer Verbindung der Formel I nach Anspruch 1, dadurch **gekennzeichnet,** daß man

(a) in einem ersten Schritt ein Cysteinderivat herstellt, indem man cin mono- oder bifunktionelles Amin oder einen mono- oder bifunktionellen Alkohol oder einen Aminoalkohol mit Cystein oder einem Derivat davon, das an der Aminfunktion und der Thiolfunktion durch eine tert.-Butyloxycarbonylgruppe geschützt ist, in Gegen wart von Dicyclohexylcarbodiimid/Hydroxybenzotriazol reagieren läßt und die tert.-Butyloxycarbonylgruppe durch Acidolyse abspaltet, und in einem zweiten Schritt

(b) ein Vanadylsulfat, aufgelöst in Wasser, unter Stickstoffatmosphare zu einer Lösung des in Stufe (a) erhaltenen Cysteinderivats in einem Gemisch aus Dimethylformamid und Boratputter bei einem pH-Wert von etwa IU in einem Verhaltnis Cystein:Vanadyl von 5:1 hinzufügt, den so erhaltenen vorstehend erwähnten Komplex gewinnt, mit Wasser wäscht und den vorstehend erwähnten Komplex trocknet.

**9.** Verfahren zur Herstellung einer Vorbindung der Formel I nach Anspruch 1, dadurch **gekennzeichnet,** daß man

- das Cystein oder ein Derivat davon mit einem Vanadylsulfat bei einem pH-Wert von etwa 7 in Wasser reagieren läßt,
- den nach Verdampfung erhaltenen Komplex gewinnt,
- den Komplex in Dimethylformamid wieder auflöst,
- weiterhin mit einem mono- oder bifunktionellen Amin, einem mono- oder bifunktionellen Alkohol oder einem Aminoalkohol über Dimethylaminopropylethylcarbodiimid als Zwischenprodukt koppelt, und
- den Komplex nach Verdampfung des Lösungsmittels in Vakuum gewinnt, und
- den Komplex mit Ether und Wasser wäscht.

**10.** Pharmazeutisches Präparat, insbesondere gegen Diabetes, dadurch **gekennzeichnet,** daß es als Wirkstoff die Verbindung nach einem der Ansprüche 1 bis 7 umfaßt.

**11.** Pharmazeutisches Präparat nach Anspruch 10, dadurch **gekennzeichnet,** daß es in fester Form, wie Kapseln, Dragees oder Analoga davon vorliegt.

**12.** Pharmazeutisches Präparat nach Anpruch 10, dadurch **gekennzeichnet,** daß es in löslicher Form, wie einer Suspension oder Tropfen oder Analoga davon vorliegt.

**13.** Pharmazeutisches Präparat nach einem der Ansprüche 10 bis 12, dadurch **gekennzeichnet,** daß es zur Herstellung eines Medikaments gegen Diabetes, das oral verabreicht werden soll, nützlich ist.

**Patentansprüche für folgende Vertragsstaaten : ES, GR**

**1.** Verfahren zur Herstellung einer organisch-anorganischen Vanadylverbindung in Form eines Komplexes aus Vanadyl und Cystein mit der folgenden allgemeinen Formel

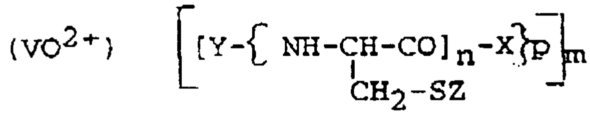

(I)

worin n, p und m jeweils ganze Zahlen mit dem Wert 1 oder 2 bedeuten, und

* in dem Falle, in dem p = 1 ist, Y für ein Wasserstoffatom, eine $-CH_2-$ oder eine R'-CO-Gruppe steht, worin R' eine Alkyl-, Aryl- oder Aralkylgruppe ist, Z eine negative Ladung oder ein Wasserstoffatom bedeutet und

(a) wenn n = 1 und m = 2 sind, X für eine $O^-$-Gruppe, eine OR-Gruppe oder eine NHR-Gruppe steht, wobei R eine Aryl-, Aralkyl- oder Alkylgruppe mit Ausschluß der Methylgruppe bedeutet, mit der Maßgabe, daß, wenn X für eine $O^-$-Gruppe steht, Z für ein Wasserstoffatom steht und Y für eine R'-CO-Gruppe steht und wenn X für eine OR- oder NHR-Gruppe steht, Z für eine negative Ladung steht und Y für eine $-CH_2$-Gruppe oder ein Wasserstoffatom steht, und

(b) wenn n = 2 und m = 1 sind, X für eine der nachstehenden Gruppen

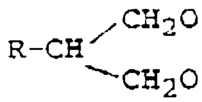

,

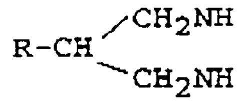

oder

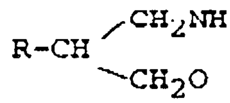

steht, wobei R eine Aryl oder Aralkylgruppe oder eine Alkylgruppe, ausgenommen eine Methylgruppe, bedeutet, und Z eine Ladung bedeutet und Y für ein Wasserstoffatom steht, und

* im Falle, in dem p = 2 ist, n = 1, m = 1, X für eine $O^-$-Gruppe steht, Z für ein Wasserstoffatom steht und Y für eine Gruppe

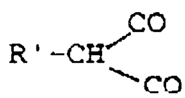

oder eine Gruppe

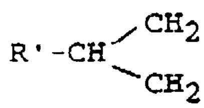

steht, wobei R' eine Alkyl-, Aryl- oder Aralkylgruppe bedeutet, dadurch **gekennzeichnet,** daß man:
(a) in einem ersten Schritt ein Cysteinderivat herstellt, indem man ein mono- oder bifunktionelles Amin oder einen mono- oder bifunktionellen Alkohol oder einen Aminoalkohol mit Cystein oder einem Derivat davon, das an der Aminfunktion und der Thiolfunktion durch eine tert.-Butyloxycarbonylgruppe geschützt ist, in Gegenwart von Dicyclohexylcarbodiimid/Hydroxybenzotriazol reagieren läßt und die tert.-Butyloxycarbonylgruppe durch Acidolyse abspaltet, und in einem zweiten Schritt
(b) ein Vanadylsulfat, aufgelöst in Wasser, unter Stickstoffatmosphäre zu einer Lösung des in Stufe (a) erhaltenen Cysteinderivats in einem Gemisch aus Dimethylformamid und Boratpuffer bei einem pH-Wert von etwa 10 in einem Verhältnis Cystein:Vanadyl von 5:1 hinzufügt, den so ernaltenen vorstehend erwähnten Komplex gewinnt, mit Wasser wäscht und den vorstehend erwähnten Komplex trocknet.

**2.** Verfahren zur Herstellung einer organisch-anorganischen Vanadylverbindung in Form eines Komplexes aus Vanadyl und Cystein der folgenden allgemeinen Formel

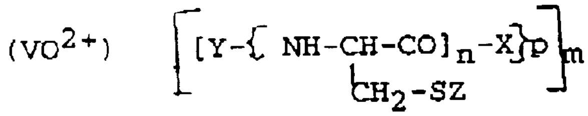

(I)

worin n, p und m jeweils für ganze Zahlen mit dem Wert 1 oder 2 stehen, und

* in dem Falle, in dem p = 1 ist, Y für ein Wasserstoffatom, eine $-CH_2$-Gruppe oder eine R'-CO-Gruppe steht, worin R' eine Alkyl-, Aryl- oder Aralkylgruppe ist, Z für eine negative Ladung oder ein Wasserstoffatom steht, und

(a) wenn n = 1 und m = 2, X für eine $O^-$-Gruppe, eine OR-Gruppe oder eine NHR-Gruppe steht, R für eine Aryl-, Aralkyl- oder Alkylgruppe, ausgenommen die Methylgruppe, steht, mit der Maßgabe, daß, wenn X für eine $O^-$ -Gruppe steht, Z für ein Wasserstoffatom steht und Y für eine R'-CO-Gruppe steht, und wenn X für eine OR-Gruppe oder eine NHR-Gruppe steht, Z für eine negative Ladung steht und Y für eine -$CH_2$-Gruppe oder ein Wasserstoffatom steht, und
(b) wenn n = 2 und m = 1, X für eine der folgenden Gruppen

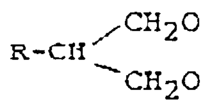
,
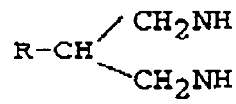

oder

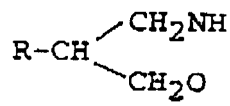

steht, wobei R eine Aryl- oder Aralkyl- oder Alkylgruppe, ausgenommen die Methylgruppe, bedeutet und Z für eine Ladung steht und Y für ein Wasserstoffatom steht, und

* in dem Falle, in dem p = 2 ist, n = 1, m = 1, X für eine $O^-$-Gruppe steht, Z für ein Wasserstoffatom steht und Y für eine Gruppe

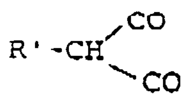

oder eine Gruppe

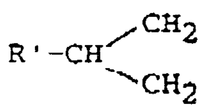

steht, wobei R' eine Alkyl-, Aryl- oder Aralkylgruppe bedeutet, dadurch **gekennzeichnet,** daß man
- das Cystein oder ein Derivat davon mit einem Vanadylsulfat bei einem pH-Wert von etwa 7 in Wasser reagieren läßt,
- den nach Verdampfung erhaltenen Komplex gewinnt,
- den Komplex in Dimethylformamid wieder auflöst, weiterhin mit einem mono- oder bifunktionellen Amin, einem mono- oder bifunktionellen Alkohol oder einem Aminoalkohol über Dimethylaminopropylethylcarbodiimid als Zwischenprodukt koppelt, und
- den Komplex nach Verdampfung des Lösungsmittels in Vakuum gewinnt, und
- den Komplex mit Ether und Wasser wäscht.

**3.** Verfahren zur Herstellung nach Anspruch 1 oder 2, dadurch **gekennzeichnet,** daß das Cystein in seiner L-Form vorliegt.

**4.** Verfahren zur Herstellung nach Anspruch 1 oder 2, dadurch **gekennzeichnet,** daß das Cystcin in seiner D-Form vorliegt.

**5.** Verfahren zur Herstellung nach einem der Ansprüche 1 bis 4, dadurch **gekennzeichnet,** daß man eine Verbindung der Formel I, worin p = 1, n = 1, m = 2, X für eine OR- oder eine NHR-Gruppe steht, worin R für eine Aryl-, Aralkyl- oder Alkylgruppe, ausgenommen die Methylgruppe, steht, erhält, wobei die Verbindung einer der folgenden Formeln II oder VI

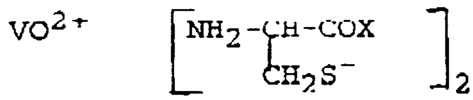

(II)

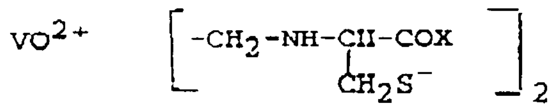

(VI)

entspricht.

**6.** Verfahren zur Herstellung nach einem der Ansprüche 1 bis 4, dadurch **gekennzeichnet,** daß man eine Verbindung der Formel I, worin p = 1, n = 1, m = 2, Y für eine R'-CO-Gruppe steht, worin R' eine Alkyl-, Aryl- oder Aralkylgruppe bedeutet, erhält, wobei die Verbindung der folgenden Formel IV entspricht

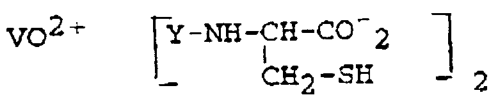

(IV)

**7.** Verfahren zur Herstellung nach einem der Ansprüche 1 bis 4, dadurch **gekennzeichnet,** daß man eine Verbindung der Formel I, worin p = 1, n = 2, m = 1 und X für eine der folgenden Gruppen

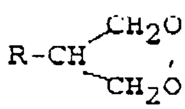 , 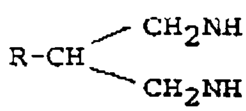

oder 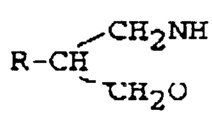

steht, worin R eine Aryl-, Aralkyl- oder Alkylgruppe, ausgenommen die Methylgruppe, bedeutet, erhält, wobei die Verbindung der folgenden Formel III entspricht

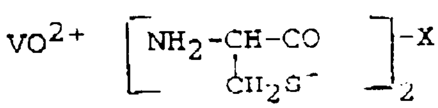

(III)

**8.** Verfahren zur Herstellung nach einem der Ansprüche 1 bis 4, dadurch gekennzeichnet, daß man eine Verbindung der Formel I, worin p = 2, n = 1, m = 1 und Y für eine Gruppe

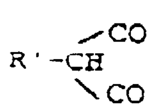

oder eine Gruppe

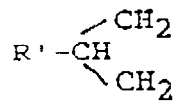

steht, worin R' eine Alkyl-, Aryl- oder Aralkylgruppe bedeutet, erhält, wobei die Verbindung der folgenden Formel V entspricht

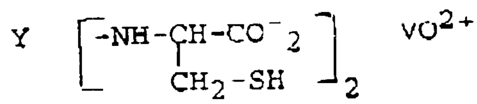

(V)

9. Verfahren zur Herstellung eines pharmazeutischen Präparats, dadurch **gekennzeichnet,** daß man der Zusammensetzung eine Verbindung, erhalten nach einem der Ansprüche 1 bis 8, hinzufügt.

10. Verfahren zur Herstellung eines Präparats nach Anspruch 9, dadurch **gekennzeichnet,** daß man das Präparat in fester Form, wie Kapseln, Dragees oder Analoga davon formuliert.

11. Verfahren zur Herstellung eines Präparats nach Anspruch 9, dadurch **gekennzeichnet,** daß man das Präparat in löslicher Form, wie einer Suspension oder Tropfen oder Analoga davon formuliert.

12. Verwendung eines Präparats, erhalten nach einem der Ansprüche 9 bis 11, zur Herstellung eines Medikaments gegen Diabetes, das oral verabreicht werden soll.

Fig. 1

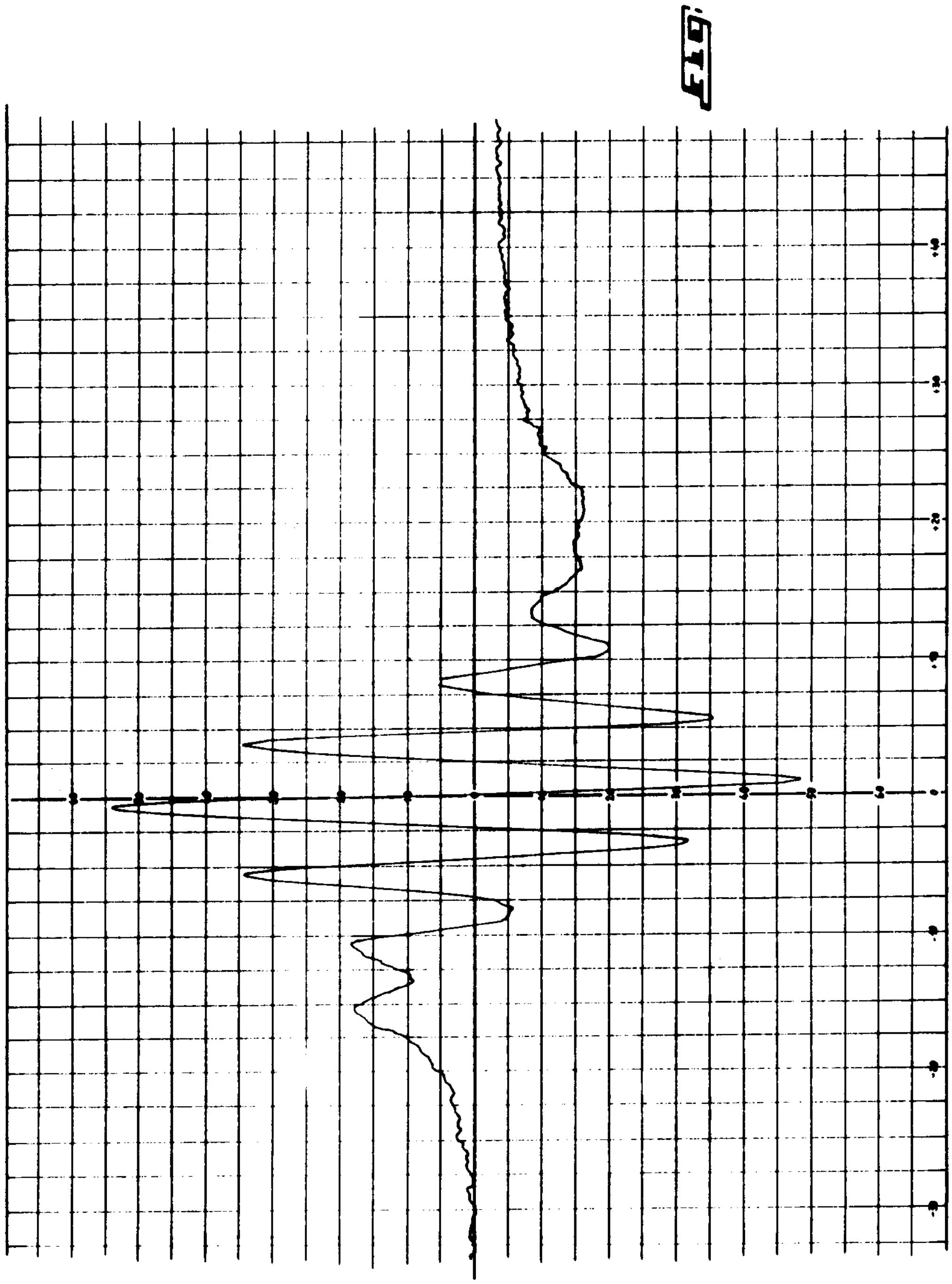

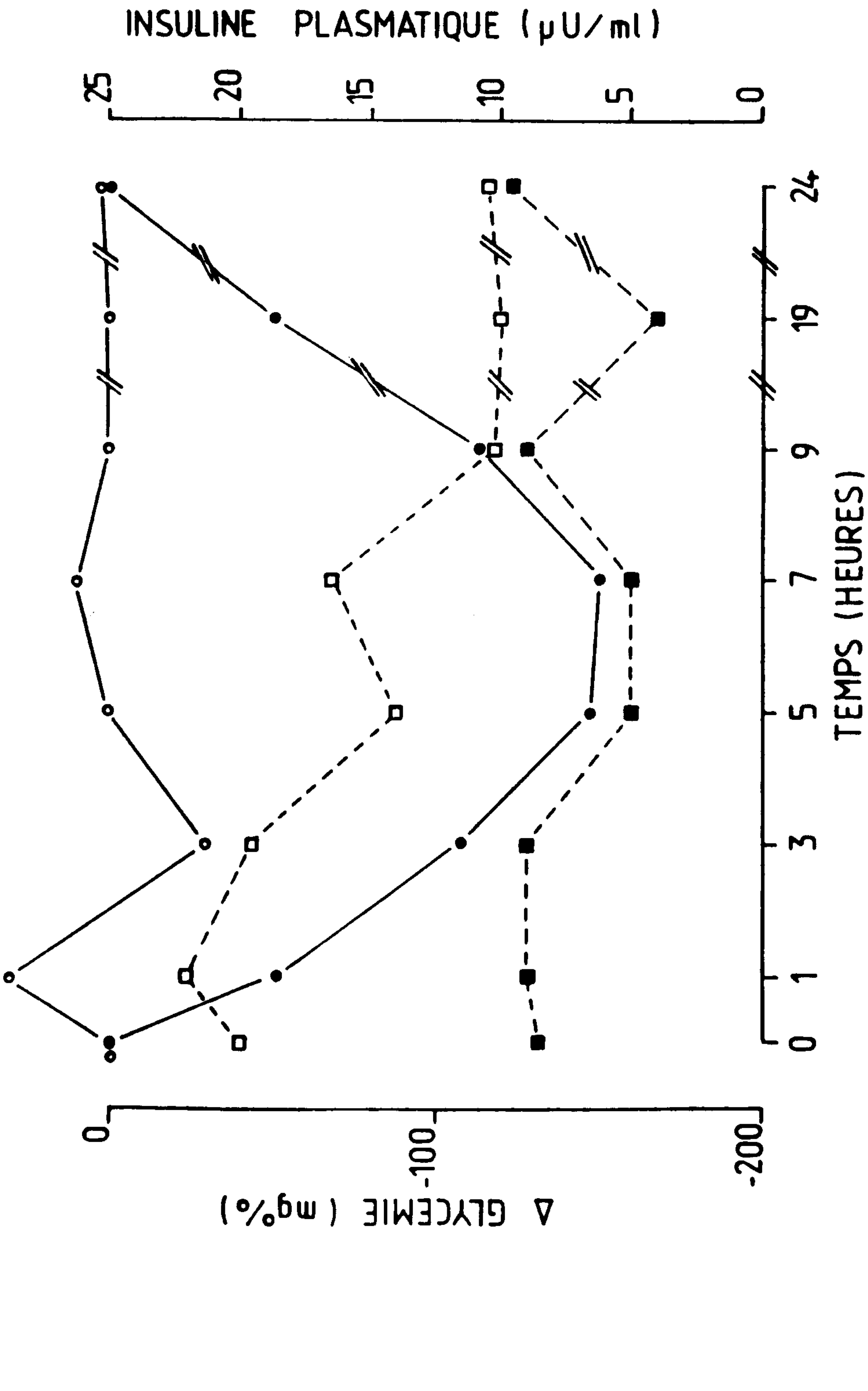
INSULINE PLASMATIQUE (µU/ml)
25
20
15
10
5
0
0
1
3
5
7
9
19
24
TEMPS (HEURES)
Δ GLYCEMIE (mg%)
0
-100
-200

Fig. 2